(19) Europäisches Patentamt
European Patent Office
Office européen des brevets

(11) Publication number: **0 007 652**
**B1**

(12) **EUROPEAN PATENT SPECIFICATION**

(45) Date of publication of patent specification: **10.02.82**

(51) Int. Cl.³: **C 07 D 209/52,**
C 07 C 93/04

(21) Application number: **79200324.6**

(22) Date of filing: **20.06.79**

(54) **Derivatives of 3-azabicyclo(3.1.0)hexane and a process for their preparation.**

(30) Priority: **27.06.78 GB 2797878**

(43) Date of publication of application:
**06.02.80 Bulletin 80/3**

(45) Publication of the grant of the European patent:
**10.02.82 Bulletin 82/6**

(84) Designated Contracting States:
**BE CH DE FR GB IT NL SE**

(56) References cited:
**DD - A - 133 943**
**DE - A1 - 2 641 295**
**DE - A1 - 2 653 251**
**US - A - 4 088 652**

(73) Proprietor: **SHELL INTERNATIONALE RESEARCH MAATSCHAPPIJ B.V.**
**Carel van Bylandtlaan 30**
**NL-2596 HR DEN HAAG (NL)**

(72) Inventor: **Day, Janet Anne**
**5, Franklin Road**
**Gillingham Kent (GB)**
Inventor: **Devlin, Barry Roy John**
**59, Ruins Barn Road**
**Sittingbourne Kent (GB)**
Inventor: **Searle, Robert John Griffith**
**Highworth Stockershill**
**Rodmersham Green Kent (GB)**

(74) Representative: **Keuzenkamp, Abraham et al,**
**c/o Shell Internationale Research Maatschappij B.V. P.O. Box 302**
**NL-2501 CH 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 007 652 B1

# Derivatives of 3-azabicyclo[3.1.0]hexane and a process for their preparation

The present invention relates to derivatives of 3-azabicyclo[3.1.0]hexane and a process for their preparation.

2-carboxy-3-azabicyclo[3.1.0]hexane and certain derivatives thereof, exhibit interesting biological properties, being capable of sterilizing male anthers in plants. In particular, German Offenlegungsschrift 2,641,295 discloses such sterilant activity for 2-carboxy-3-azabicyclo[3.1.0]hexane and its 2-methoxy derivative. The bicyclic ring structure of these compounds is, however, very difficult to synthesize.

According to F. Chem. Soc., 2087—2093 (1959) 4-amino-1,1-diethoxybutane can be converted into 2-cyano-pyrrolidine using potassium cyanide and hydrochloric acid. A yield of 29% was reported. When the Applicants reacted cis-2-methylaminocyclopropane-1,1-diethylacetal with potassium cyanide in the presence of hydrochloric acid the formation of a bicyclic compound containing a ring-nitrogen atom was not observed.

It has now been found that certain novel derivatives of 3-azabicyclo[3.1.0]hexane can be prepared in good yields from the corresponding 2-amino-alkylcyclopropyl acetals, and used in the preparation of biologically active compounds.

The present invention provides a compound of the general formula:

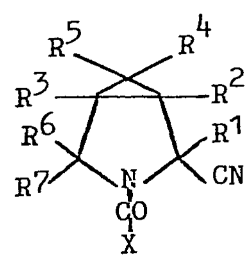

(I)

wherein $R^1$ represents a hydrogen atom or an alkyl group of up to 6 carbon atoms, which may be substituted by one or two halogen atoms and/or alkoxy groups having up to 4 carbon atoms; $R^2$, $R^3$, $R^4$, $R^5$, $R^6$ and $R^7$ each independently represents a hydrogen atom or a halogen atom or an alkyl or alkenyl group of up to 6 carbon atoms which may contain up to 3 halogen atoms, or an aryl, alkaryl or aralkyl group of up to 10 carbon atoms, which may contain up to 3 halogen atoms; or $R^4$ and $R^5$ or $R^6$ and $R^7$ together form an alkylene moiety of up to 6 methylene groups; and X represents an alkyl group having up to 5 carbon atoms. Preferably, each of $R^1$ to $R^7$ represents a hydrogen atom, and preferably X represents an ethyl group.

The invention also provides a process for the preparation of a compound of the general formula I, which comprises reacting a compound of the general formula:

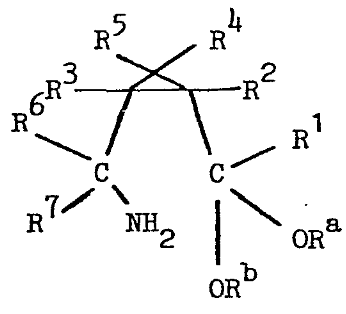

(II)

wherein $R^a$ and $R^b$ each independently represents an alkyl group of up to 4 carbon atoms, $R^1$, $R^2$, $R^3$, $R^4$, $R^5$, $R^6$ and $R^7$ have the meanings given for the general formula I and the $R_6R_7CNH_2$ group has a cis-relationship with the group —$CR^1(OR^a)(OR^b)$, with a compound of formula M—CN, wherein M represents a hydrogen atom or an alkali metal or one equivalent of an alkaline earth metal or an ammonium moiety, in the presence of an alkane carboxylic acid having up to 5 carbon atoms.

A preferred embodiment of the process according to the present invention is the preparation of 2-cyano-3-acetyl-3-azabicyclo[3.1.0]hexane by reacting cis-2-methylamino cyclopropane-1,1-diethylacetal with potassium cyanide in the presence of acetic acid.

The process according to the present invention will normally produce a mixture of geometrical and/or optical isomers. If desired, the isomers obtained may be separated by methods known in the art.

Convenient starting materials in the process according to the present invention are compounds according to formula II wherein $R^1$ represents a hydrogen atom or an alkyl group of up to 4 carbon atoms, e.g., a methyl group; $R^2$, $R^3$, $R^4$, $R^5$, $R^6$ and $R^7$ each independently represents a hydrogen, fluorine, chlorine or bromine atom or an alkyl or alkenyl group of up to 4 carbon atoms, e.g., a methyl or

isobutenyl group, which group(s) may contain up to 3 fluorine, chlorine or bromine atoms, e.g., a difluoro- or dichlorovinyl group; or $R^4$ and $R^5$ together form an alkylene moiety of 4 or 5 methylene groups; and $R^a$ and $R^b$ each independently represents a methyl- or an ethyl group. Cis-2-methylamino-cyclopropane-1,1-diethylacetal is a suitable starting material.

The compounds according to formula II are reacted with a cyanide compound in the presence of an alkane carboxylic acid of up to 5 carbon atoms. Suitable cyanides include hydrogen cyanide, alkali metal cyanides, such as sodium or potassium cyanide, and ammonium or alkyl-substituted ammonium cyanides, such as tri- or tetramethyl ammonium cyanide. If hydrogen cyanide is used, this may be generated *in situ*. Examples of compounds which can generate hydrogen cyanide *in situ* are aldehyde and ketone cyanohydrins, such as acetone cyanohydrin, methyl ethyl ketone cyanohydrin and acetaldehyde cyanohydrin.

The process according to the present invention is conveniently carried out using an alkali metal cyanide as the cyanide source. Preference is given to the use of sodium or potassium cyanide. Good results are generally obtained when the cyanide is used in slight molar excess, e.g., up to 10% by weight based on the cyclopropyl derivative according to formula II.

The process according to the present invention is conveniently carried out using an excess of the alkane carboxylic acid. This acid may also, if desired, serve as a solvent for the reaction mixture. Preference is given to the use of glacial acetic acid in the process according to the invention.

The reaction between the compound according to formula II and the compound of formula M—CN is suitably carried out at ambient or moderately elevated temperatures. Temperatures up to the boiling point of the reaction medium may be used, preference being given to temperatures in the range of from 40°C to 120°C.

A small amount of an alkyl or arylsulphonic acid, such as methanesulphonic acid or *p*-toluene sulphonic acid, may also be present in the reaction mixture as it has a catalytic effect on the reaction. The amount of the sulphonic acid is preferably in the range 0.3—10% by weight, preferably 2—6% by weight, based on the cyclopropyl derivative of formula II. If desired, a small amount of a mineral acid, for example hydrochloric acid, may be present in the reaction mixture.

The process according to the present invention is most conveniently carried out at atmospheric pressure. If required, superatmospheric pressures, e.g. pressures up to 10 atmospheres, may be applied.

The process according to the present invention may be carried out in the presence of a solvent, for example, an ether such as diethyl ether or tetrahydrofuran, or an aliphatic nitrile such as acetonitrile. An excess if the alkane carboxylic acid reactant may be used as solvent or co-solvent.

The compound of the general formula I produced by the process according to the invention, may be isolated from the reaction mixture by any suitable method, or it may be further reacted *in situ* to produce a compound of the general formula:

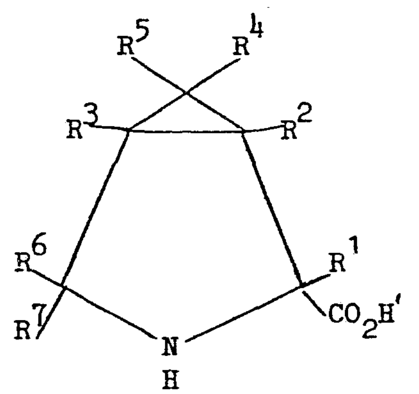

(III)

in which $R^1$—$R^7$ have the meanings given above; or a salt and/or ester thereof. Compounds of the general formula III and salts and/or esters thereof have valuable biological properties.

The invention therefore also provides a process for the preparation of a compound of the general formula III or a salt and/or an ester thereof, characterized in that a compound of the general formula I is hydrolyzed and/or alcoholized.

A free acid of the general formula III or an acid addition salt thereof may be prepared by treating a compound of the general formula I with a strong acid, for example hydrochloric acid. Esters of the free acid or acid addition salts may be prepared by reaction of a compound of the general formula I with an alcohol in the presence of an acid catalyst; for example reaction with ethanol in the presence of dry hydrogen chloride leads to an ethyl ester. Preferred esters are those derived from alkanols having up to 6 carbon atoms.

The compounds according to formula II are believed to be novel compounds, and may be prepared by methods analogous to methods known in the art. For example, 2-methylaminocyclopropyl-1,1-diethylacetal can conveniently be prepared by converting ethyl 2-cyanocyclopropane

carboxylate (see Zhur. Org. Khim., 7, No. 10, 2108 (1971)) into 2-cyanocyclopropyl-1,1-diethylacetal which compound is then treated with a reducing agent, such as lithium aluminium hydride.

The invention is illustrated by the following Example.

## Example

### Preparation of 2-carboxy-3-azabicyclo[3.1.0]hexane

(a) Preparation of 2-cyanocyclopropyl-1,1-diethylacetal

Cis-ethyl 2-cyanocyclopropylcarboxylate (34.5 g; 0.25 mol.) in water (120 ml) was treated very slowly with sodium hydroxide (9.9 g) in water (248 ml). The reaction mixture was washed with diethyl ether, acidified with concentrated hydrochloric acid and 2-cyanocyclopropyl carboxylic acid (21.3 g) was obtained in 77% yield.

27 ml oxalylchloride were added dropwise to 2-cyanocyclopropyl carboxylic acid (13.5 g; 0.12 mol.) in dry benzene (60 ml) at 70—80°C. The reaction mixture was kept under reflux for two hours and then evaporated until dry. The crude product obtained was dissolved in dry tetrahydrofuran (100 ml) and treated with a solution of tri-tert. butoxy lithium aluminium hydride at —60°C in dry tetrahydrofuran (150 ml). After stirring the reaction mixture for two hours at —60°C it was poured onto ice and extracted with chloroform. Evaporation of chloroform from the extract gave 2-cyanocyclopropylaldehyde (8.4 g) in 74% yield. The structure was confirmed by infra-red and NMR spectroscopy.

2-cyanocyclopropylaldehyde (8.1 g; 0.09 mol.) in ethanol (75 ml) was refluxed under stirring for two hours under a Dean Stark trap. The solution was then evaporated and 2-cyanocyclopropyl-1,1-diethylacetal was obtained by distillation at 70—71°C/8 mm; 5.2 g of product were obtained in 34% yield.

| *Analysis* | C | H | N |
|---|---|---|---|
| Calculated for $C_9H_{15}O_2N$: | 63.9 | 8.9 | 8.3% |
| Found: | 63.6 | 9.1 | 8.5% |

(b) Preparation of 2-methylaminocyclopropyl-1,1-diethylacetal

2-cyanocyclopropyl-1,1-diethylacetal (9 g; 0.05 mol.), prepared according to (a) in dry tetrahydrofuran (90 ml) was added to lithium aluminium hydride (2.3 g) in dry tetrahydrofuran (250 ml) at room temperature. After stirring the reaction mixture for 48 hours at ambient temperature sodium hydroxide (45 ml of 10% solution) was added. The liquid was decanted and extracted with ether. After extraction of ether the desired product was obtained (8.5 g). The product was characterized by infra-red (liquid film): 3360 $cm^{-1}$ (NH); 1110 $cm^{-1}$ (C—O—C); and NMR in $CDCl_3$: (ppm): 1.4 (m,4H); 1.8 (5,6H); 2.4 (s,2H); 3.2 (t,2H); 4.2 (m,4H) and 4.8 (t,1H).

(c) Preparation of 2-cyano-3-acetyl-3-azabicyclo[3.1.0]hexane

2-methylaminocyclopropyl-1,1-diethylacetal (2 g, 11.5 mmol.) was added to potassium cyanide (6.8 g) in glacial acetic acid (90 ml) and methanesulphonic acid (0.2 ml) at 40°C. The mixture was kept at a temperature of 65°C during 17 hours and then poured onto ice. After extraction 1.1 g of 2-cyano-3-acetyl-3-azabicyclo[3.1.0]hexane was obtained. Yield (without further purification) 57%.

(d) Preparation of 2-carboxy-3-azabicyclo[3.1.0]hexane

0.95 g of 2-cyano-3-acetyl-3-azabicyclo[3.1.0]hexane was dissolved in 6 N hydrochloric acid (about 100 ml) and refluxed over 6 hours. The hydrochloric acid salt of cis-2-carboxy-3-azabicyclo[3.1.0]hexane was obtained in an amount of 1.25 g. Pure cis-2-carboxy-3-azabicyclo[3.1.0]hexane was obtained by treating the hydrochloric acid salt with copper(II) hydroxide and extracting the copper(II) salt formed with methanol followed by conversion of the salt into the final product, which had NMR characteristics consistent with published data.

## Claims

1. A compound of the general formula:

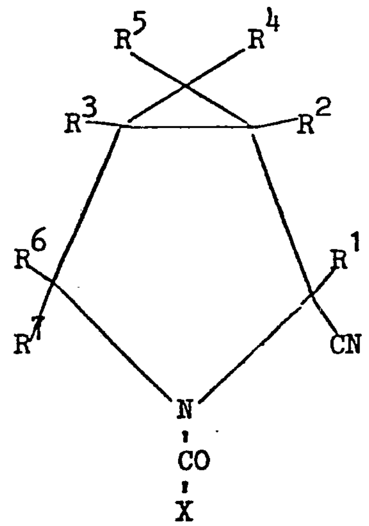

(I)

wherein $R^1$ represents a hydrogen atom or an alkyl group of up to 6 carbon atoms, which may be substituted by one or two halogen atoms and/or alkoxy groups having up to 4 carbon atoms; $R^2$, $R^3$, $R^4$, $R^5$, $R^6$ and $R^7$ each independently represents a hydrogen or a halogen atom or an alkyl or alkenyl group of up to 6 carbon atoms which may contain up to 3 halogen atoms, or an aryl, alkaryl or aralkyl group of up to 10 carbon atoms, which may contain up to 3 halogen atoms; or $R^4$ and $R^5$ or $R^6$ and $R^7$ together form an alkylene moiety of up to 6 methylene groups; and X represents an alkyl group having up to 5 carbon atoms.

2. A compound as claimed in claim 1, in which each of $R^1$ to $R^7$ represents a hydrogen atom.

3. A compound as claimed in either claim 1 or claim 2, in which X represents an ethyl group.

4. A process for the preparation of a compound as claimed in any one of claims 1 to 3, characterized in that it comprises reacting a compound of the general formula:

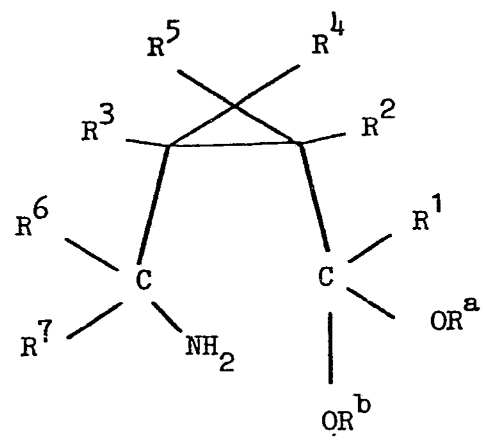

(II)

wherein $R^a$ and $R^b$ each independently represents an alkyl group of up to 4 carbon atoms, $R^1$, $R^2$, $R^3$, $R^4$, $R^5$, $R^6$ and $R^7$ have the meanings given for the general formula I and the $R_6R_7CNH_2$ group has a cis-relationship with the group $-CR^1(OR^a)(OR^b)$, with a compound of formula M—CN, wherein M represents a hydrogen atom or an alkali metal or one equivalent of an alkaline earth metal or an ammonium moiety, in the presence of an alkane carboxylic acid having up to 5 carbon atoms.

5. A process as claimed in claim 4, characterized in that in the compound of the general formula II, each of $R^a$ and $R^b$ independently represents a methyl or an ethyl group.

6. A process as claimed in either claim 4 or claim 5, characterized in that a cyanide is used in which M represents an alkali metal.

7. A process as claimed in claim 4, characterized in that the alkane carboxylic acid used is acetic acid.

8. A process as claimed in any one of claims 4 to 7, characterized in that it is conducted at a temperature in the range of from 40 to 120°C.

9. A process for the preparation of a compound of the general formula:

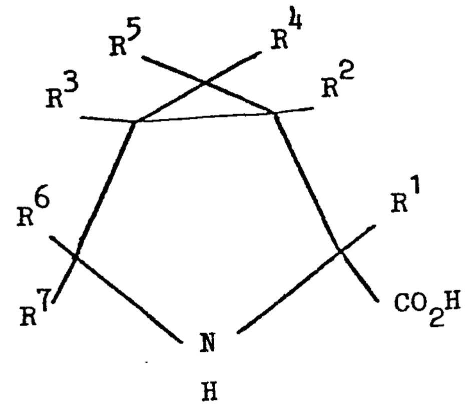

in which R¹—R⁷ have the meanings given in claim 1, or a salt and/or an ester thereof, characterized in that a compound as claimed in claim 1 is hydrolyzed and/or alcoholized.

**Patentansprüche**

1. Verbindung der allgemeinen Formel:

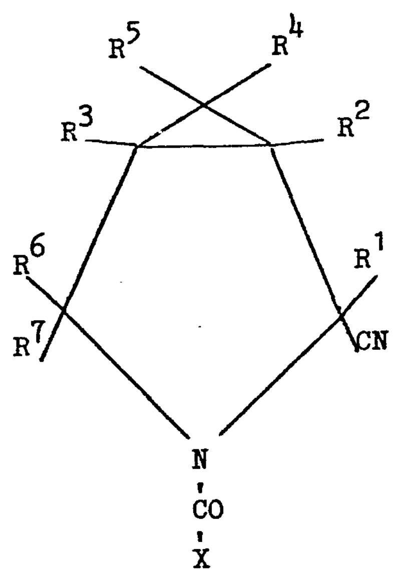

(I)

in der $R^1$ ein Wasserstoffatom oder eine Alkylgruppe mit bis zu 6 Kohlenstoffatomen bedeutet, die durch ein oder zwei Halogenatome und/oder Alkoxygruppen mit bis zu 4 Kohlenstoffatomen substituiert sein kann; $R^2$, $R^3$, $R^4$, $R^5$, $R^6$ und $R^7$ jeweils unabhängig voneinander ein Wasserstoff- oder Halogenatom oder eine Alkyl- oder Alkenylgruppe mit bis zu 6 Kohlenstoffatomen, die bis zu 3 Halogenatome enthalten kann, oder eine Aryl-, Alkaryl- oder Aralkylgruppe mit bis zu 10 Kohlenstoffatomen, die bis zu 3 Halogenatome enthalten kann; oder $R^4$ und $R^5$ oder $R^6$ und $R^7$ zusammen eine Alkylengruppe mit bis zu 6 Methylengruppen bilden; und X eine Alkylgruppe mit bis zu 5 Kohlenstoffatomen bedeutet.

2. Verbindung nach Anspruch 1, wobei jeder der Reste $R^1$ bis $R^7$ ein Wasserstoffatom bedeutet.

3. Verbindung nach Anspruch 1 oder 2, wobei X eine Äthylgruppe bedeutet.

4. Verfahren zur Herstellung einer Verbindung nach einem der Ansprüche 1 bis 3, dadurch gekennzeichnet, dass man eine Verbindung der allgemeinen Formel:

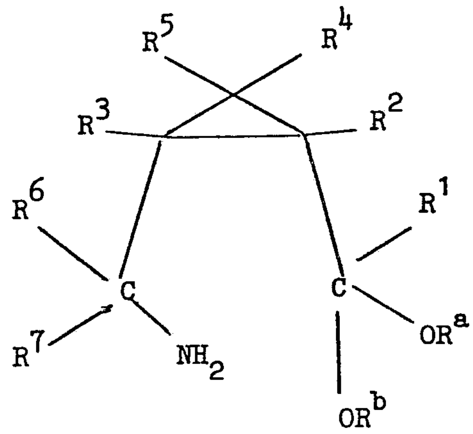

(II)

in der $R^a$ und $R^b$ jeweils unabhängig voneinander eine Alkylgruppe mit bis zu 4 Kohlenstoffatomen bedeuten, $R^1$, $R^2$, $R^3$, $R^4$, $R^5$, $R^6$ und $R^7$ die für die allgemeine Formel I angegebene Bedeutung haben und die Gruppe $R^6R^7CNH_2$ sich in cis-Stellung zu der Gruppe $-CR^1(OR^a)(OR^b)$ befindet, umsetzt mit einer Verbindung der Formel M—CN, wobei M ein Wasserstoffatom oder ein Alkalimetallatom oder ein Äquivalent eines Erdalkalimetalls oder eine Ammoniumgruppe bedeutet, in Gegenwart einer Alkancarbonsäure mit bis zu 5 Kohlenstoffatomen.

5. Verfahren nach Anspruch 4, dadurch gekennzeichnet, dass bei der Verbindung der allgemeinen Formel II $R^a$ und $R^b$ jeweils unabhängig voneinander eine Methyl- oder eine Äthylgruppe bedeuten.

6. Verfahren nach Anspruch 4 oder 5, dadurch gekennzeichnet, dass ein Cyanid angewandt wird, bei dem M ein Alkalimetallatom bedeutet.

7. Verfahren nach Anspruch 4, dadurch gekennzeichnet, dass die angewandte Alkancarbonsäure Essigsäure ist.

8. Verfahren nach einem der Ansprüche 4 bis 7, dadurch gekennzeichnet, dass es bei einer Temperatur im Bereich von 40 bis 120°C durchgeführt wird.

9. Verfahren zur Herstellung einer Verbindung der allgemeinen Formel:

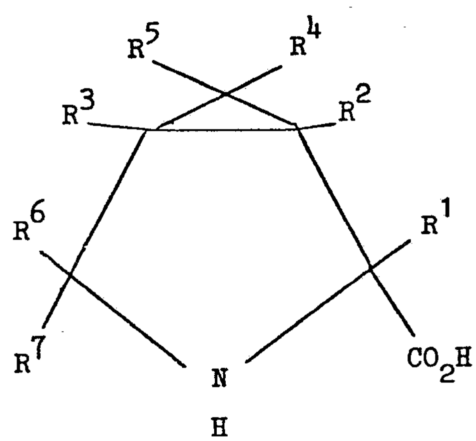

in der $R^1$ bis $R^7$ die in Anspruch 1 angegebene Bedeutung haben oder eines Salzes und/oder eines Esters davon, dadurch gekennzeichnet, dass eine Verbindung nach Anspruch 1 hydrolysiert und/oder alkoholisiert wird.

**Revendications**

1. Composé de la formule générale:

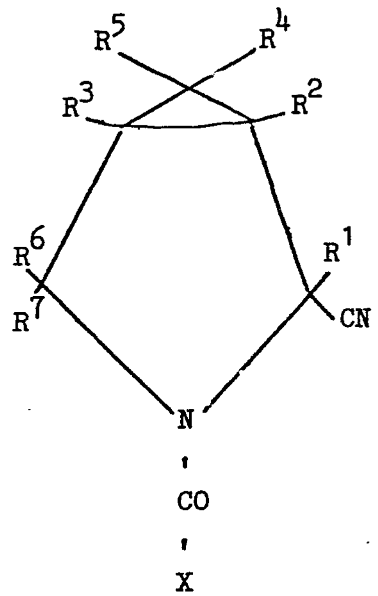

(I)

dans laquelle $R^1$ représente un atome d'hydrogène ou un groupe alcoyle ayant jusqu'à 6 atomes de carbone, qui peut être substitué par un ou deux atomes d'halogènes et/ou groupes alcoxy ayant jusqu'à 4 atomes de carbone; $R^2$, $R^3$, $R^4$, $R^5$, $R^6$ et $R^7$ représentent chacun indépendamment un atome d'hydrogène ou d'un halogène ou un groupe alcoyle ou alcényle ayant jusqu'à 6 atomes de carbone qui peut contenir jusqu'à 3 atomes d'halogènes, ou un groupe aryle, alcaryle ou aralcoyle ayant jusqu'à 10 atomes de carbone, qui peut contenir jusqu'à 3 atomes d'halogènes; ou $R^4$ et $R^5$ ou $R^6$ et $R^7$ forment ensemble une portion alcoylène de jusqu'à 6 groupes méthylène; et X représente un groupe alcoyle ayant jusqu'à 5 atomes de carbone.

2. Composé selon la revendication 1, caractérisé en ce que chacun des R de $R^1$ à $R^7$ représente un atome d'hydrogène.

3. Composé selon l'une des revendications 1 et 2, caractérisé en ce que X représente un groupe éthyle.

4. Procédé pour la préparation d'un composé tel que revendiqué dans l'une quelconque des revendications 1 à 3, caractérisé en ce qu'on fait réagir un composé de la formule générale:

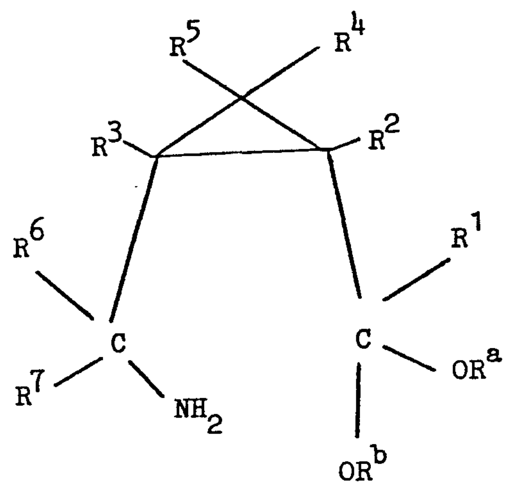

(II)

dans laquelle $R^a$ et $R^b$ représentent chacun indépendamment un groupe alcoyle ayant jusqu'à 4 atomes de carbone, $R^1$, $R^2$, $R^3$, $R^4$, $R^5$, $R^6$ et $R^7$ ont les significations indiquées pour la formule générale I et le groupe $R^6R^7CNH_2$ a une relation cis avec le group $-CR^1(OR^a)(OR^b)$, avec un composé de formule M—CN, dans laquelle M représente un atome d'hydrogène ou d'un métal alcalin ou un équivalent d'un métal alcalinoterreux ou une portion ammonium, en présence d'un acide alcanecarboxylique ayant jusqu'à 5 atomes de carbone.

5. Procédé selon la revendication 4, caractérisé en ce que dans le composé de la formule générale II, $R^a$ et $R^b$ représentent chacun indépendamment un groupe méthyle ou éthyle.

6. Procédé selon l'une des revendications 4 et 5, caractérisé en ce qu'on utilise un cyanure dans lequel M représente un métal alcalin.

7. Procédé selon la revendication 4, caractérisé en ce que l'acide alcanecarboxylique utilisé est l'acide acétique.

8. Procédé selon l'une quelconque des revendications 4 à 7, caractérisé en ce qu'il est mis en oeuvre à une température comprise entre 40 et 120°C.

9. Procédé pour la préparation d'un composé de la formule générale:

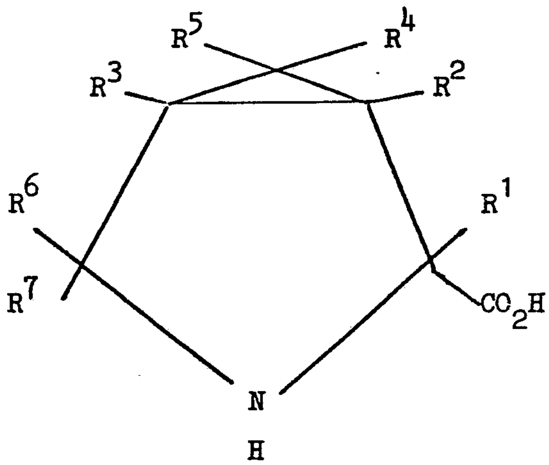

dans laquelle les R de $R^1$ à $R^7$ ont les significations indiquées dans la revendication 1, ou d'un sel et/ou d'un ester d'un tel composé, caractérisé en ce qu'un composé tel que revendiqué dans la revendication 1 est hydrolysé et/ou alcoolysé.